# EUROPEAN PATENT SPECIFICATION

(11) **EP 2 338 473 B1**
(45) Date of publication and mention of the grant of the patent: **13.07.2016**
(21) Application number: 09425528.8
(22) Date of filing: 18.12.2009
(51) Int. Cl.: A61K 9/08, A61K 31/433

(54) **Pharmaceutical dosage forms of tizanidine and administration route thereof**
Pharmazeutische Dosierungsformen von Tizanidin und Verabreichungsweg dafür
Formes galéniques pharmaceutiques de tizanidine et voie d'administration associée

(43) Date of publication of application: 29.06.2011
(73) Proprietor: MDM S.p.A., I-20123 Milano (IT)
(72) Inventor: Borsa, Massimiliano, 20190 Vimodrone/ MI (IT)
(74) Representative: Beneduce, Gianna

(56) References cited:
- EP-A1- 2 135 601
- WO-A1-2004/043431
- YURIKA KINO ET AL: "Involvement of Supraspinal Imidazoline Receptors and Descending Monoaminergic Pathways in Tizanidine-Induced Inhibition of Rat Spinal Reflexes" JOURNAL OF PHARMACOLOGICAL SCIENCES, JAPANESE PHARMACOLOGICAL SOCIETY, TOKYO, JP LNKD- DOI:10.1254/JPHS.FP0050520, vol. 99, no. 1, 1 January 2005 (2005-01-01), pages 52-60, XP008122407 ISSN: 1347-8613 [retrieved on 2005-08-26]
- DATABASE CA [Online] CHEMICAL ABSTRACTS SERVICE, COLUMBUS, OHIO, US; 15 September 2009 (2009-09-15), CHEN, GANG ET AL: "Tizanidine hydrochloride and tandospirone citrate combined medicine for treating headache" XP002582463 retrieved from STN Database accession no. 151:389366 & CN 101 530 413 A (CHENGDU LUKAI PHARMACEUTICAL SCIENCE AND TECHNOLOGY CO., LTD., PEOP. R) 16 September 2009 (2009-09-16)
- KAWAMATA TOMOYUKI ET AL: "Antihyperalgesic and Side Effects of Intrathecal Clonidine and Tizanidine in a Rat Model of Neuropathic Pain" ANESTHESIOLOGY, AMERICAN SOCIETY OF ANESTHESIOLOGISTS, PHILADELPHIA, PA, US, vol. 98, no. 6, 1 June 2003 (2003-06-01), pages 1480-1483, XP008122410 ISSN: 0003-3022
- DATABASE CA [Online] CHEMICAL ABSTRACTS SERVICE, COLUMBUS, OHIO, US; 27 November 2008 (2008-11-27), NIMISHA, M. BAJPAI ET AL: "Formulation and evaluation of bioadhesive microspheres of tizanidine hydrochloride for nasal drug delivery" XP002582464 retrieved from STN Database accession no. 149:518832 & INDIAN PHARMACIST (NEW DELHI, INDIA) , 7(74), 71-72, 75-77 CODEN: IPNHA9; ISSN: 0972-7914, 2008,

## Description

### State of the art

Tizanidine, 5-chloro-N-(4,5-dihydro-1H-imidazol-2-yl)-2,1,3-benzothia-diazol-4-amine, is a α₂-adrenergic agonist structurally related to clonidine. It is a centrally acting skeletal muscle relaxant that acts mainly at spinal and supraspinal levels to inhibit excitatory interneurones. It is indicated for the symptomatic relief of spasticity associated with multiple sclerosis or spinal cord injury or disease or painful muscle spasm.

The compound tizanidine, as hydrochloride, is included in Japan and US Pharmacopoeias and it is approved and marketed worldwide, as base, in the form of 2 or 4 mg tablets. As such, tizanidine is usually well and rapidly absorbed by oral route, but it undergoes extensive first-pass effect which inactivates metabolites. The pharmacokinetic profile of tizanidine, Tₘₐₓ 1₋2h, T_{1/2z} 1-2h, suggested to perform slow-release dosage forms as in US 2008 0214629A1 or WO 2008 047208A1, but, on the other hand, the entity of the first-pass effect, suggested to replace oral absorption with different routes of administration.

Are known in the art patent documents which describe pharmaceutical formulations containing tizanidine: trans-dermal formulations are described in DK 175982B1, buccal spray formulations are described in WO 2004 019905A1, buccal and sublingual formulations are described in WO 2004 043431A1 and in US 2004 0122065A1. The aim of these documents is to increase the onset of action of tizanidine as well as its bioavailability and to reduce the relative hepatic first-pass effect.

Pharmacological papers reported the administration of tizanidine by intramuscular (1M) or intrathecal route to assess its mechanism of action and compare its potency with standard drugs (J. Neurosurg 2004, 101(4):641-7; J. Pharmacol Sci. 2005, 99(1):52-60; Anesthesiology 2003, 98(6):1480-3; Anesth. Analg. 2003, 96(3):776-82; Anesth.

Analg. 2001, 93(5):1310).

In EP-2014305 liquid intranasal compositions are suggested with muscle relaxant in a long list of actives.

### Description of the invention

It is the object of the present invention a pharmaceutical composition in liquid dosage form which contains as active principle the α₂-adrenergic agonist compound Tizanidine.

According to the invention it has been found that tizanidine, in the form of the corresponding hydrochloride, is soluble in water at concentrations and pH suitable for systemic administration, specifically for intranasal administration, and that these aqueous solutions of tizanidine hydrochloride may be normally stored and, in addition, easily sterilized by heat so that the use of preservative agents can be, when preferred, avoided.

Pharmaceutical compositions of tizanidine in liquid form, such as the aqueous solutions of the present invention, offer different advantages: they can be administered by intranasal route (IN) to increase the bioavailability overcoming the hepatic first-pass effect and/or to shorten the time to peak. The transmucosal nasal delivery, represents a delivery option for drugs with limited oral bioavailability due to the degradation in the intestinal tract, such as proteins, or hepatic first-pass metabolism, and is also a convenient alternative to intravenous or intramuscular drug administration. The considerable blood flow, actually responsible for breath conditioning, benefits from the efficient systemic drug uptake and provides direct access to the systemic circulation for transmucosal adsorbed drug.

In accordance with the administration route used, the composition of the invention may also contain suitable, commonly used agents such as pH buffer, preservative, flavouring, absorption enhancer and hyperbaric agents.

Among commonly used suitable pH buffer agents, aqueous buffer acetate and aqueous buffer phosphate solutions are preferred. Among the commonly used preservative agents, benzyl alcohol, methyl-, ethyl- and propylparahydroxybenzoate are preferred. Among commonly used water soluble flavouring agents citrus, preferably orange, eucalyptol, eucalyptus oil and peppermint are preferred. Among commonly used absorption enhancer agents chitosan, methylpyrrolidone and cholic acid are preferred. Suitable tonicity of solutions may be obtained by addition of saline or salt solutions.

The amount of tizanidine, as base, contained in the composition of the invention, which can be daily administered to a patient, may vary in a large range and depends on various factors, such as the pathology to be treated, the intranasal administration relative bioavailability, the age and conditions of the patient.

The daily dose of tizanidine, as base, is generally in the range 6.00 -12.00 mg/day. The pH value of the intranasal aqueous pharmaceutical composition may vary from 4.8 to 7.4. Tizanidine intranasal administrations may contain absorption enhancer agents such as chitosan, methylpyrrolidone or cholic acid and preservative agents such as benzyl alcohol, methyl-, ethyl- and propylparahydroxybenzoate or similar products and flavouring compounds as citrus, preferably orange, eucalyptol, eucalyptus oil or peppermint aroma and similar products.

The compositions containing tizanidine hydrochloride aqueous solutions of the present invention, that is the compositions suitable for intranasal administration, proved to be well tolerated at the administration site, and showed themselves to be effective for relief both of muscle spasm and of multiple sclerosis and neuronal spasticity. They can be administered over a wide range of doses according to the pathology, bioavailability, and peak time.

Detailed formulations and physico-chemical properties are hereinafter provided.

### Intranasal solutions

Aqueous solutions, with or without flavour, at 22.90 mg/mL suitable to be dispensed as two 50 µL puff by 0.05 mL snap-on pump, equal to 2 mg of tizanidine base. Aqueous solutions, with or without flavour, at 45.80 mg/mL suitable to be dispensed as two 50 µL puff by 0.05 mL snap-on pump, equal to 4.00 mg of tizanidine base.

### Preparation of composition of intranasal spray solutions

An aqueous solution of tizanidine.HCl at high concentration, 45.00 mg/mL, and pH 4.8 is tolerated by the nasal mucosa without any local side effect, i.e. redness or pain. The proposed formulations are calculated for a single puff of 50 µL/nostril.

### Example 1

| | |
|---|---|
| Tizanidine.HCl | 22.90 mg |
| Methyl p-hydroxybenzoate | 1.00 mg |
| Eucalyptol | 4.00 mg |
| 95% Ethanol | 0.40 mL |
| Water to | 1.00 mL |

Yellow, clear solution, pH 4.8

The formulation allows administering 1.00 or 2.00 mg of tizanidine base with 1 or 2 puffs.

### Example 2

| | |
|---|---|
| Tizanidine.HCl | 45.80 mg |
| Methyl p-hydroxybenzoate | 1.00 mg |
| Eucalyptol | 4.00 mg |
| 95% Ethanol | 0.40 mL |
| Water to | 1.00 mL |

Yellow, clear solution, pH 4.8

The formulation allows administering 2.00 or 4:00 mg of tizanidine base with 1 or 2 puffs.

### Example 3

| | |
|---|---|
| Tizanidine.HCl | 22.90 mg |
| Benzyl alcohol | 10.00 mg |
| Eucalytptol | 4.00 mg |
| Phosphate buffer pH 7.4 (0.03M) to | 1.00 mL |
| Yellow, clear solution, pH 7.4 | |

The formulation allows administering 1.00 or 2.00 mg of tizanidine base with 1 or 2 puffs.

### Example 4

| | |
|---|---|
| Tizanidine.HCl | 22.90 mg |
| Benzyl alcohol | 10.00 mg |
| Eucalytptol | 4.00 mg |
| Chitosan.HCl | 10.00 mg |
| Phosphate buffer pH 7.4(0.03M) to | 1.00 mL |

Yellow, clear solution, pH 7.4

The formulation allows administering 1.00 or 2.00 mg of tizanidine base with 1 or 2 puffs.

### Example 5

| | |
|---|---|
| Tizanidine.HCl | 22.90 mg |
| Benzyl alcohol | 10.00 mg |
| Eucalytptol | 4.00 mg |
| Cholic acid | 14.00 mg |
| Phosphate buffer pH 7.4(0.03M) to | 1.00 mL |

Yellow, clear solution, pH 7.4.

The formulation allows administering 1.00 or 2.00 mg of tizanidine base with 1 or 2 puffs.

### Pharmacokinetics of tizanidine solutions

Preliminary pharmacokinetic data in rabbits at the dose of 3.00 mg/kg of tizanidine hydrochloride by intranasal (IN), intramuscular (IM), and oral route (PO) were performed to test the intranasal absorption without any enhancer. Tizanidine (IN) is rapidly absorbed with a bioavailability close to that of intramuscular route (IM).

## Claims

1. A pharmaceutical composition of tizanidine in liquid form for use in therapy by intranasal administration, **characterized by** the fact that it contains the active ingredient, in its hydrocloride form, dissolved in an aqueous solution and buffer solution at a pH value selected in the range from 4.8 to 7.4, that the obtained solution, duly sterilized by a heat treatment or added with a suitable preservative agent, may be administered as such or, optionally, be added with one or more suitable commonly used flavouring, tonicity, hyperbaric and absorption enhancer agents.

2. The pharmaceutical composition of tizanidine in liquid form according to claim 1, **characterized by** the fact that the sterilization is carried out by a heat treatment.

3. The pharmaceutical composition of tizanidine in liquid form according to claim 1 or 2, **characterized by** the fact that the preservative agent is selected in the group consisting of benzyl alcohol, methylparahydroxybenzoate, cthylparahydroxybcnzoate and propylparahydroxybenzoate, that the flavouring agent is selected from the group of aroma consisting of citrus, eucalyptol, eucalyptus oil and peppermint optionally in the presence of a tonicity agent and/ or hyperbaric agent

4. The pharmaceutical composition of tizanidine in liquid form for intranasal spray administration according to any of claim 1 to 3, **characterized by** the fact that it contains tizanidine at the concentration from 20.00 to 40.00 mg/mL in the presence of an absorption enhancer agent selected in the group consisting of chitosan. methylpyrrolidone and cholic acid.

5. The pharmaceutical composition of tizanidine in liquid form according to claim 4, **characterized by** the fact that the absorption enhancer agent is chitosan.

## Patentansprüche

1. Pharmazeutische Zusammensetzung aus Tizanidin in flüssiger. Form zur Verwendung in der Therapie durch intranasale Verabreichung, **dadurch gekennzeichnet, dass** sie den Wirkstoff in seiner hydrochloriden Form in einer wässrigen Lösung und Pufferlösung mit einem pH-Wert im Bereich zwischen 4,8 und 7,4 gelöst enthält, dass die erhaltene Lösung, die ordnungsgemäß durch eine Erhitzung oder ein Hinzufügen eines geeigneten Konservierungsmittels sterilisiert ist, als solche verabreicht oder wahlweise mit einem oder mehreren geeigneten, üblicherweise verwendete Geschmacks-, Tonizitäts- hyperbaren und absorptionsverbessernden Mitteln versehen werden kann.

2. Pharmazeutische Zusammensetzung aus Tizanidin in flüssiger Form nach Anspruch 1, **dadurch gekennzeichnet, dass** die Sterilisierung durch eine Erhitzung durchgeführt wird.

3. Pharmazeutische Zusammensetzung aus Tizanidin in flüssiger Form nach Anspruch 1 oder 2, **dadurch gekennzeichnet, dass** das Konservierungsmittel aus der Gruppe ausgewählt wird, die aus Benzylakohol, Methylparahydroxybenzoat, Äthylparahydroxybenzoat und Propylparahydroxybenzoat besteht, und dass das Geschmacksmittel aus der Gruppe von Aromen ausgewählt wird, die aus Zitrus, Eukalyptus, Eukalyptusöl und Pfefferminze besteht; wobei wahlweise ein Tonizitätsmittel und/oder hyperbares Mittel beigegeben ist.

4. Pharmazeutische Zusammensetzung aus Tizanidin' in flüssiger Form zur intranasalen Verabreichung durch Sprühen nach einem der Ansprüche 1 bis 3, **dadurch gekennzeichnet, dass** sie Tizanidin in einer Konzentration zwischen 20,00 bis 40,00 mg/mL enthält, wobei ein absorptionsverbesserndes Mittel beigegeben ist, das aus der Gruppe ausgewählt wird, die aus Chitosan, Methylpyrrolidon und Cholsäure besteht.

5. Pharmazeutische Zusammensetzung aus Tizanidin in flüssiger Form nach Anspruch 4, **dadurch gekennzeichnet, dass** das absorptionsverbessernde Mittel Chitosan ist.

## Revendications

1. Composition pharmaceutique de tizanidine en forme liquide pour l'usage en thérapie par administration intranasal, **caractérisée par le fait qu'**elle contient l'ingrédient actif, en sa forme hydrochlorydrate, dissous dans une solution aqueuse et une solution tampon à une valeur de pH choisi entre 4,8 et 7,4, que la solution obtenue, dûment stérilisée par traitement thermique ou par addition d'un ou plusieurs aptes agents conservatifs, peut être administrée telle quelle ou, facultativement, avec l'addition d'un ou plusieurs aptes agents communément utilisés pour aromatiser, donner tonicité, augmenter l'hyperbaricité et l'absorption.

2. Composition pharmaceutique de tizanidine en forme liquide selon la revendication 1, charactérisée par le fait que la stérilisation a lieu par traitement termique.

3. Composition pharmaceutique de tizanidine en forme liquide selon la revendication 1 ou 2, charactérisée par le fait que l'agent conservatif est choisi dans le groupe consistant de alcool benzylique, méthylparahydroxybenzoate, éthylparahydroxybenzoate et propylparahydroxybenzoate, que l'agent aromatisant est choisi dans le groupe d'arômes consistants des agrumes, eucalyptol, huile d'eucaliptol et menthol facultativement en présence d'un agent de tonicité et/ou d'un agent d'hyperbaricité.

4. Composition pharmaceutique de tizanidine en forme liquide pour administration de spray nasal selon une quelconque des revendications 1 à 3, charactérisée par le fait qu'elle contient tizanidine à la concentration de 20,00 à 40,00 mg/mL en présence d'un agent augmentatif de l'absorption choisi dans le groupe consistant en chitosane, méthylpyrrholidon et acide cholique.

5. Composition pharmaceutique de tizanidine en forme liquide selon la revendication 4, charactérisée par le fait que l'agent augmentatif de l'absorption est chitosane.
